# EUROPEAN PATENT APPLICATION

(11) **EP 2 974 725 A1**
(43) Date of publication of application: **20.01.2016**
(21) Application number: 15176602.9
(22) Date of filing: 14.07.2015
(51) Int. Cl.: A61K 31/4045, A61P 1/02, A61K 9/00, A61K 9/06, A61K 31/155, A61P 29/00, A61K 36/53

(54) **PHARMACEUTICAL COMPOSITION**

(30) Priority: 16.07.2014 IT MI20141295
(71) Applicant: D'Alfonso, Luca, 71016 San Severo (IT)
(72) Inventor: D'Alfonso, Luca, 71016 San Severo (IT)
(74) Representative: Mittler, Enrico

(57) **Abstract**

The present invention relates to a pharmaceutical composition comprising melatonin and chlorhexidine.

The present invention also relates to a use of such a pharmaceutical composition in the preparation of a medicament for treating and/or preventing mucositis.

## Description

### Field of the invention

The present invention relates to a particular pharmaceutical composition comprising melatonin (N-acetyl-5-methoxytryptamine) or salts or derivatives thereof and chlorhexidine or salts or derivatives thereof for treatment and/or prevention of mucositis.

### Background art

Melatonin is released into the saliva and can play a vital role in protecting the mucous membranes of the mouth and nearby structures from damage caused, in the broadest sense, from oxidative stress. Oxidative stress refers to the damages caused by free radicals of oxygen and nitrogen. For example, the singlet oxygen (oxygen free radical), having a non-paired radical in its outer orbital, in order to achieve stability tends to subtract it from the first molecule with which it will come into contact. On a molecular basis, the oxidative stress causes damage that affects also tissues and organs, amplifying itself. The mouth is one of the organs most burdened by this kind of oxidative stress.

Nowadays, the treatment of malignant tumors with radiotherapy and/or chemotherapy is increasingly effective but is associated with short and long term side effects. Among these side effects are oral mucosal function disorders; the main consequences are severe ulcers (mucositis) and mouth fungal superinfection (candidiasis, thrush).

Mucositis is one of the oral complications affecting 30-40% of patients receiving chemo-radio therapy and up to 80% of patients undergoing hematopoietic stem cell transplantation. It was also found that about 97% of patients suffering from cancer of the head and neck develop some degree of mucositis, as well as 100% of patients undergoing fractionated radiation therapy for a prolonged time (Trotti A. et al. Radioterapia e Oncologia 2003, 66:253-262).

These complications caused by the disease and its treatments involve pain in swallowing, dysphagia, malnutrition, delay in the administration of chemotherapy, radiotherapy system interruptions, loss of effectiveness of cancer treatments, long hospitalization, high costs and in some patients, potentially fatal infections.

Mucositis is an inflammatory reaction that affects the entire gastrointestinal tract, from the mouth to the anus, and is one of the major side effects of chemotherapy and/or radiotherapy and bone marrow transplantation.

Mucositis can also be caused by chemical agents such as corticosteroids, immunosuppressants (azathioprine, Cyclosporine A), drugs that cause xerostomia, anxiolytics, antidepressants, antihistamines, sympathomimetics stimulants, antiparkinsonians, antipsychotics, gum treatments, idantoine or broad-spectrum antibiotics.

Currently, drug treatments are intended to merely control symptoms and prevent complications. Cryotherapy, analgesics, opioids and antifungals are used with variable results.

Patent application EP 2,702,992-A1 discloses the use of a composition comprising melatonin at a concentration in the range of 2.5%-5% by weight per volume in 100 ml of final composition to prepare a pharmaceutical composition for treating and/or preventing mucositis. This document also teaches that concentrations lower than the range indicated above, thus less than 2.5% by weight per volume, cannot fully reverse mucositis.

However, the Applicant has found that concentrations of melatonin in amounts exceeding 2.5% by weight per volume, as shown in 2,702,992 EP-A1, produce unwanted effects, such as falling asleep or tachycardia and depression, particularly frequent, particularly in the presence of high doses of melatonin. Among other less common side effects are psychosis, movement disorders, hypertension, anxiety, dry mouth, weakness, yellow eyes and skin caused by a change in the composition of blood. Other side effects, though rare, are an increase in sexual desire, mood changes, reduction of platelets, blood sodium decreased, fainting and night cramps.

Therefore, there is the need to identify a pharmaceutical composition that allows eliminating or at least reducing the generation of disorders such as mucositis or other disorders of the oral cavity, without inducing effects in the patient, such as the tendency to fall asleep.

### Summary of the invention

In a first aspect thereof, the present invention relates to a pharmaceutical composition as the one indicated in claim 1.

In fact, the Applicant of the present application has surprisingly found that a pharmaceutical composition comprising a compound of general formula (I): 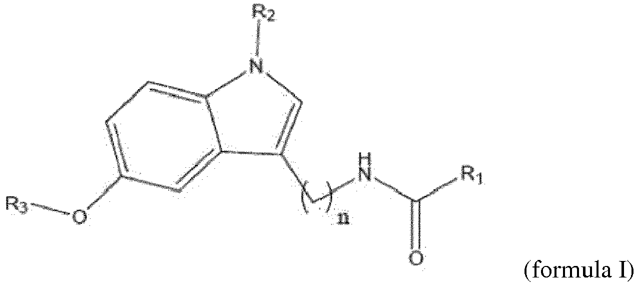 wherein n is an integer between 1 and 4;

R₁ and R₃, equal or different, represent a C₁-C₄ linear or branched alkyl group; and
R₂ is selected from hydrogen, a C₁-C₄ linear or branched alkyl group, a -C(=O) O-Ra group or a -C(=O)-N(H)-Ra group, wherein Ra is a C₁-C₄ linear or branched alkyl group,
or salts, prodrugs or solvates of such a formula,
wherein the concentration of said compound is less than 2.5% by weight per volume in 100 ml of said pharmaceutical composition,
and comprising chlorhexidine, or salts or derivatives thereof, in a concentration in the range between 0.10% and 0.20% by weight per volume in 100 ml of said pharmaceutical composition,
is able to reduce/eliminate the generation of disorders such as mucositis or other disorders of the oral cavity.

In fact, through the application of the pharmaceutical composition of the present invention, wherein there is the compound of general formula (I) with a concentration lower than 2.5% by weight per volume and chlorhexidine in a concentration in the range between 0.10% and 0.20% by weight per volume, a particular benefit was found in patients suffering from mucositis without inducing at the same time toxicity or unwanted side effects, such as falling asleep or tachycardia and depression, particularly frequent, especially in the presence of high doses of said compound by formula (I).

Therefore, the Applicant has found that concentrations of the compound of general formula (I) lower than 2.5% by weight per volume and chlorhexidine in a concentration in the range between 0.10% and 0.20% by weight per volume, in contrast to the teachings provided by 2.702.992 EP-A1, where it is stated that such a compound must be present with a concentration from 2.5% to 5% by weight per volume, are useful to solve the technical problem mentioned above.

In the present invention, the term "pharmaceutical composition" or "drug" refers to any substance used to prevent, diagnose, alleviate, treat or cure diseases in humans or animals.

In the present invention, the term "prodrug" refers to biologically inactive molecules which, when introduced into the body, undergo physical and chemical changes by enzymes, most liver enzymes, which activate the molecules. The term "prodrug" therefore defines a class of molecules that are precursors of active ingredients.

In the present invention, the term "alkyl" refers to aliphatic chains having from 1 to 4 carbon atoms, for example methyl, ethyl, n-propyl, i-propyl, n-butyl, tert-butyl, branched or linear sec-butyl, n-pentyl, etc.

Preferably, in the compound of general formula (I) above, R₁ and R₃, equal or different, are a C₁-C₂ alkyl group, more preferably a methyl group.

Preferably, in the compound of general formula (I) above, R₂ is hydrogen and n=1.

Preferably, the compound of general formula (I) above is melatonin.

The term "melatonin" refers to N-acetyl-5-methoxytryptamine, also known in literature as N-[2-(5-methoxy-1H-indol-3-yl)ethyl]acetamide with CAS registry number 73-31-4.

Melatonin is an neuro-endogenous hormone that is physiologically produced in animals, including humans, by the pineal gland (epiphysis cerebri) located at the base of the brain and by other organs, such as the gastrointestinal tract, the retina, lymphocytes and bone marrow cells.

Melatonin is produced in animals, including humans, from serotonin (5-hydroxytryptamine, 5-HT), which in turn is derived from tryptophan. Melatonin is also produced in plants.

Preferably, the concentration of said compound of general formula (I) is in the range 1.8% -2.2%, more preferably about 2% by weight per volume of said pharmaceutical composition.

In this way, the pharmaceutical composition of the present invention acts more efficiently on the hypothalamus, thus ensuring better regulation of the sleep-wake cycle, while too high concentrations would compromise the proper use of the product.

Chlorhexidine is known for its broad-spectrum antiseptic action, both against Gram + and Gram - and fungi. Its bactericidal action is due to an increase in the membrane permeability with alteration of the protein structure. This causes the precipitation of several cytoplasmic macromolecules with death by lysis of bacterial and fungal cells.

Preferably, the pharmaceutical composition of the present invention includes chlorhexidine, or salts or derivatives thereof, e.g., chlorhexidine benzalkonium chloride, chlorhexidine benzododecinium chloride, chlorhexidine dodecyldimonium chloride, chlorhexidine sodium fluoride, chlorhexidine diacetate, chlorhexidine dihydrochloride, chlorhexidine digluconate and the like.

In this way, a synergy of action of melatonin with chlorhexidine or a salt or derivative thereof is created on different fronts of mucositis, generally of mouth and mouth appendages disorders. In fact, chlorhexidine or a salt or derivative thereof increases the access channels to the endogenous action of melatonin and such activity is particularly useful in counteract the evolution of certain forms of cancer in the oral cavity. Moreover, an indirect re-epithelizing activity of "*restitutio in integrum*" of the mucosa is obtained as an effect due to the synergistic activity of "reclamation" towards the inflammatory and infectious damage ("*noxae*").

Preferably, the pharmaceutical composition of the present invention comprises said chlorhexidine or a salt or derivative thereof at a concentration from 0.01% to 0.50% by weight, more preferably from about 0.10% to about 0.20%, per volume of 100 ml of said pharmaceutical composition.

Preferably, the pharmaceutical composition of the present invention comprises from about 1.8% to about 2.2% by weight, preferably about 2.0% by weight of melatonin and from about 0.10% to about 0.20% by weight, preferably about 0.12% by weight of chlorhexidine or a salt or derivative thereof per volume of 100 ml of said pharmaceutical composition.

Preferably, the pharmaceutical composition of the present invention further comprises a gelling agent.

In the present invention, the term "gelling" refers to a substance which forms a gel, i.e., a three-dimensional mesh formed by the gelling agent, and generally it contains a liquid phase.

Preferably, the gelling agent is selected from the list comprising vinyl polymers, cross-linked polyacrylate polymers, polyethylene and polypropylene copolymers, cellulose and guar gum. For example, copolymers called Pluronic^{R} such as the Pluronic^{R} F127 or Pluronic^{R} F127NF copolymer, or vinyl polymers called carbopol, such as Carbomer 910, 934, 940, 941, may be used as gelling agents.

Preferably, the pharmaceutical composition of the present invention comprises a gelling agent with a concentration from about 0.1% to about 2% by weight, more preferably from about 0.5% to about 1.0% by weight.

Preferably, the pharmaceutical composition of the present invention is in gel form (also referred to as a "hydrogel"); preferably, the pharmaceutical composition of the present invention comprises melatonin and chlorhexidine or a salt or derivative thereof and is in gel form.

Its gelification is made through the dispersion of the gelling agent in water, thus creating a slightly acidic environment, which subsequently is neutralized in order to make the pH basic.

Preferably, the pharmaceutical composition of the present invention comprises a pH balancer, such as sodium hydroxide, potassium hydroxide or triethanolamine, preferably with concentration from about 0.1% to about 2.0% by weight, preferably from about 0.5% to about 1.0% by weight.

In this way, a high concentration of active agents in a pathological suffering area may be obtained. In fact, the administration of a gel that comprises melatonin and chlorhexidine or a salt or derivative thereof allows a drastic reduction of free radicals, such as oxygen and nitrogen, originated during the inflammation, as well as an increase of 2-type interleukin, of interferon gamma and of CD4⁺ cell populations with consequent reduction of the infective charge in the mouth.

Preferably, the pharmaceutical composition of the present invention further comprises at least one pharmaceutically acceptable excipient or adjuvant.

In the present invention, the term "excipient" refers to a substance which promotes the absorption of the medicinal or pharmaceutical composition of the invention, stabilizes it or contributes to its preparation, that is, giving consistency to the pharmaceutical composition providing such flavors as to make it more palatable. Therefore, excipients can have the function of retaining the ingredients such as starches, sugars or celluloses; the softening function, to act as dye, protect the drug so as to isolate it from the air and/or humidity, as a filler for a pill, capsule or any other form of presentation, of disintegration to facilitate the dissolution of the components and their absorption in the intestine, without excluding other-type excipients not mentioned in this paragraph.

Examples of excipients useful for the objects of the present invention are, for example, sodium lauryl sulphate, polyethylene glycol (PEG) 4000, 6000, castor oil, hydrogenated vegetable oils, methylcellulose, gelatin, etc.

Preferably, the pharmaceutical composition of the present invention comprises said excipients with a concentration in the range between 0.1% and 20%, more preferably between 0.2% and 10% by weight per volume.

Preferably, the pharmaceutical composition of the present invention comprises natural substances such as plant extracts or oils derived therefrom, alone or in combination.

In this way, a further synergistic effect can be obtained, provided by the combination of melatonin, and at least one of such natural substances on the mucous membranes of the oral cavity.

Preferably, the pharmaceutical composition of the present invention comprises an essential lavender oil. In this way, properties are obtained that relax the central nervous system and the involuntary muscles; in fact, the linalool found in lavender essential oil, in combination with melatonin and preferably with chlorhexidine, blocks the neurostimulating action of glutamate, a known exciting neurotransmitter, and increases the levels of GABA (gamma-aminobutyric acid), a major inhibitory neurotransmitter of the central nervous system.

Preferably, the pharmaceutical composition of the present invention comprises a compound of general formula (I), preferably melatonin, in a concentration lower than or equal to 2% by weight per volume, chlorhexidine in a concentration in the range between 0.10% and 0.20% by weight per volume and an essential lavender oil, preferably with a concentration in the range between 0.1% and 5%, more preferably between 1% and 2% by weight per volume.

In this way, thanks to the presence in the pharmaceutical composition of the present invention of an essential lavender oil which is able to cross the blood-brain barrier, it is possible to further reduce the amount of the compound of general formula (I) preferably melatonin, obtaining the same beneficial effects to treat and/or prevent mucositis, further reducing the side effects.

Preferably, the pharmaceutical composition of the present invention comprises extracts from the flowering tops of Hypericum perforatum, herbaceous plant of the Hypericum genus, rich in hyperine, a flavonoid useful for soothing, anti-inflammatory, healing and re-epithelizing properties.

Preferably, the pharmaceutical composition of the present invention comprises said Hypericum perforatum extracts with a concentration in the range between 0.1% and 5%, more preferably between 0.5% and 3% by weight per volume.

The high concentration of tannins, characteristic of freeze-dued extracts, exhibits excellent astringent properties, useful in the treatment of oily and impure skins.

Preferably, the pharmaceutical composition of the present invention comprises Calendula extracts, rich in triterpenes, flavonoids, polysaccharides, carotenes and phytosterols.

Preferably, the pharmaceutical composition of the present invention comprises said Calendula officinalis flower extract with a concentration in the range between 0.1% and 5%, more preferably between 0.5% and 3% by weight per volume.

In this way, it is possible to obtain remarkable soothing properties to attribute to the component consisting of saponifiable flavonoids and mucilage and remarkable immunostimulatory capabilites due to the polysaccharide component.

In particular, the active ingredients contained in the extract of calendula, particularly flavonoids, exert an anti-inflammatory effect on sensitive and inflamed skin, stimulate the re-epithelization, accelerate the epidermal turnover and promote the activity of skin fibroblasts, promoting the collagen synthesis.

Preferably, the pharmaceutical composition of the present invention comprises as extracts essential oil of Melaleuca alternifolia, also known as tea tree, a tree of the Mirtaceae family.

Preferably, the pharmaceutical composition of the present invention comprises said Melaleuca alternifolia extracts with a concentration in the range between 0.1% and 5%, more preferably between 0.5% and 1% by weight per volume.

In this way, antiseptic and antifungal benefits are obtained, as well as antibacterial, antiviral and pain relief of canker sores and bleeding gingivitis.

Preferably, the pharmaceutical composition of the present invention comprises Argan oil, preferably with a concentration in the range between 0.1% and 10%, more preferably between 1% and 5% by weight per volume.

Argan oil, being rich in vitamins A and F, as well as flavonoids, xanthophylls, carotenoids, sterols and triterpenes, is useful for quick absorption and for giving a soft feel to the buccal mucosa.

In this way, advantages are obtained from the viewpoint of softening and reddening properties.

Preferably, the pharmaceutical composition of the present invention comprises sunflower oil, preferably with a concentration in the range between 0.1% and 10%, more preferably between 1% and 5% by weight per volume.

Sunflower seed oil is particularly rich in vitamins (among the highest in the plants world) such as vit. B12, vit. B1, the fat-soluble vitamins A, D and E, vit. PP and niacin.

Preferably, the pharmaceutical composition of the present invention comprises at least an essence, such as cinnamon, lemon, orange, mandarin orange or vanilla, or combinations thereof, preferably with a concentration in the range between 0.01% and 5%, more preferably between 0.02% and 2% by weight per volume.

In this way, the composition of the invention is able to take a pleasant taste.

In the present invention, the term "adjuvant" refers to any substance which improves the response of a drug substance; it may refer to any adjuvant known by the man skilled in the art.

In the present invention, the term "pharmaceutically acceptable" refers to the compound of formula (I) of the pharmaceutical composition of the present invention which is such as not to damage the bodies in which it is administered.

Preferably, the pharmaceutical composition of the present invention also comprises at least one preservative, where "preservative" means a substance that retains the properties of the drug product by inhibiting germ contamination. Preferably, said preservative is a ionic or non-ionic preservative.

Preferably, said preservative is non-toxic, chemically stable and compatible with melatonin.

Examples of preservatives useful for the objects of the present invention are, for example, benzoic acid, sodium benzoate, ascorbic acid, potassium sorbate, methyl paraben, ethylparaben or butylparaben.

Preferably, the pharmaceutical composition of the present invention comprises at least one of said preservatives with a concentration in the range between 0.01% and 2%, more preferably between 0.1% and 0.6% by weight per volume.

In the present invention, the term "germs" refers to any cell that can grow and multiply in the pharmaceutical composition of the invention, e.g. bacteria, fungi and yeasts.

Preferably, the pharmaceutical composition of the present invention further comprises at least one antioxidant agent.

In the present invention, the term "antioxidant agent" refers to the substance that can delay or prevent oxidation.

Examples of antioxidant agents useful for the objects of the present invention are, for example, tocopherol, ascorbic acid, sodium ascorbate, tartaric acid, butylated hydroxyanisole, citric acid, vitamin A or vitamin E.

Preferably, the pharmaceutical composition of the present invention further comprises a pharmaceutically acceptable carrier.

In the present invention, the term "pharmaceutically acceptable carrier" refers to those substances, or combinations of substances, known in the pharmaceutical field used in the preparation of pharmaceutical dosage forms and include, but are not limited to, solids, liquids, solvents or surfactants.

Preferably, said vehicle is an inert substance or has an action similar to any of the compounds of the present invention.

The function of the carrier is to facilitate the insertion of the product of general formula (I) in the pharmaceutical composition of the present invention, as well as other compounds, to allow a better dosage and administration or to give form and consistency to the pharmaceutical composition.

Examples of pharmaceutically acceptable carriers useful for the objects of the present invention are, for example, lysosomes, microcapsules, nanocapsules, sponges, microspheres, nanospheres, microparticles and nanoparticles.

Preferably, the pharmaceutical composition of the present invention comprises water or other aqueous solution, preferably between 40% and 95%, more preferably between 50% and 80% by weight per volume.

In the present invention, the term "salt" refers to pharmaceutically acceptable salts of the compound of formula (I) or derivatives thereof that can be generated by chemical methods known by the man skilled in the art, for example by a reaction with an acid in water or in an organic solvent or in a mixture of the two.

Examples of organic solvents useful for the objects of the present invention are, for example, ether, ethyl acetate, ethanol, isopropanol or acetonitrile.

Examples of addition salts with acids useful for the objects of the present invention are, for example, addition salts of mineral acids such as, for example, hydrochloride, hydrobromide, hydroiodide, sulphate, nitrate, phosphate, and addition salts of organic acids such as, for example, acetate, maleate, fumarate, citrate, oxalate, succinate, tartrate, maleate, mandelate, methanesulfonate and p-toluenesulfonate.

In the present invention, the term "solvate" refers to that derivative of the compound of formula (I) to obtain another molecule, for example a polar solvent, linked by a covalent bond.

Examples of solvates useful for the objects of the present invention are, for example, hydrates and alcoholates, for example methanolates.

Salts, solvates and prodrugs can be prepared by methods known in the art.

Pharmaceutically non-acceptable solvates, prodrugs and salts are included in the scope of the invention as they can be useful in the preparation of pharmaceutically acceptable solvates, salts or prodrugs.

In the present invention, the term "comprises" and/or "includes" and variations thereof do not seek to exclude other technical features, additives, components.

In a second aspect thereof, the present invention relates to the use of a pharmaceutical composition like that indicated in claim 10.

In fact, the Applicant of the present application has surprisingly found that the use of a pharmaceutical composition as described according to the first aspect of the present invention in the preparation of a medicament for treating and/or preventing mucositis is able to reduce/eliminate the generation of disorders such as mucositis or other oral diseases.

In fact, through the application of the pharmaceutical composition of the present invention, wherein there is the compound of general formula (I) with a concentration lower than 2.5% by weight per volume and chlorhexidine in a concentration in the range between 0.10% and 0.20% by weight per volume, a particular benefit was found in patients suffering from mucositis without inducing at the same time toxicity or unwanted side effects, such as falling asleep.

The present invention also relates to the use of a composition comprising any precursor of melatonin (5-HT, tryptophan or intermediate metabolites such as N-acetylserotonin, or NAS), at a sufficient concentration so that such precursors of melatonin are converted into melatonin in the human body at the concentrations described in the present invention and chlorhexidine in a concentration of between 0.10% and 0.20% by weight per volume, for preparing a pharmaceutical composition for treating and/or preventing mucositis.

The pharmaceutical composition of the invention may be formulated for administration in a variety of forms known in the art.

Preferably, the use of said pharmaceutical composition of the present invention is in a dosage form suitable for topical, oral, intraperitoneal, intradermal or subcutaneous administration; more preferably, for topical or oral administration.

Examples of formulations which can be used in the pharmaceutical composition of the invention in the case of topical administration are for example oil in water emulsions, water in oil emulsions, milks, lotions, gels, ointments, balms, foams, body oils, soaps, vaporizers, creams, ointments, salves, and the like.

Preferably, said composition may also be incorporated in solid supports selected from the group consisting of hydrogels, wipes, patches and face masks.

The dosage to obtain a therapeutically effective amount depends on a variety of factors, such as age, weight, sex or tolerance of the animal, preferably mammal, and more preferably human, for example.

In the present invention, the term "therapeutically effective amount" refers to the pharmaceutically effective amount of the composition which produces the desired effect, and will generally be determined, among the others, by the typical characteristics of said pharmaceutical composition and the therapeutic effect sought.

A preferred embodiment of the invention relates to the use in which the daily dose administered is between 37.5 mg and 75 mg, more preferably about 45 mg, even more preferably the use in which the dose is administered in a regime of 15 mg 3 times a day.

In the present invention, the term "treat and/or prevent" refers to both therapeutic and prophylactic treatment and to preventive measures.

Preferably, the use of said pharmaceutical composition of the present invention relates to the use in which mucositis is caused by radiotherapy and/or chemotherapy.

In the present invention, the term "radiotherapy" refers to a treatment based on the use of ionizing radiation capable of ionizing the material, such as X-rays or radioactivity, for example, which includes both gamma rays and alpha particles.

In the present invention, the term "chemotherapy" refers to a treatment based on the administration of an agent which causes the inhibition of tumor growth and includes any treatment known by the man skilled in the art that generates mucositis.

Preferably, the use of said pharmaceutical composition of the present invention is for treating and/or preventing oral, pharynx, esophagus, stomach mucositis or intestinal mucositis; preferably, for treating and/or preventing oral mucositis.

For the man skilled in the art, other objects, advantages and features of the invention will be partly deduced from the description and partly from the practice of the invention.

The following examples are provided for illustrative purposes and are not intended to limit the present invention.

### Examples

### Example 1 (invention)

50mL gel samples of of the pharmaceutical composition of the invention (defined as "composition 1") were prepared as follows.

Peg-40 castor oil (10%), melatonin (2%), sunflower seed oil (6%), argan oil (5%), Melaleuca alternifolia oil (1%), Calendula officinalis flower extract (1.5%), Hypericum perforatum extract (1.5%), potassium sorbate (0.5%), lemon peel oil (0.5%) were mixed together, the percentages being by weight per volume. This mixture was gelled by adding the carbomer gelling agent 940 (1%) (a cross-linked vinyl polymer available for example from CHEMOS GmbH, Regenstauf, Germany) and dispersing all in water (70%), thus creating a slightly acidic environment, which was subsequently neutralized with the addition of tuethanolamine (1%) as a balancer in order to make the pH basic. The gel composition thus obtained was defined as composition 1.

### Example 2 (invention)

Similar to what described in Example 1, 50mL gel samples of the pharmaceutical composition of the invention (defined as "composition 2") were prepared, totally similar to composition 1 of the invention described above, with the difference that composition 2 further included 0.12% by weight per volume of Chlorhexidine digluconate, replacing a similar percentage by weight of water.

### Example 3 (invention)

Similar to what described in Example 2, 50mL gel samples of the pharmaceutical composition of the invention (defined as "composition 3") were prepared, totally similar to composition 2 of the invention described above, with the difference that composition 3 further included 0.2% by weight per volume of essential lavender oil, replacing a similar percentage by weight of melatonin, which therefore was in an amount of 1.8% by weight per volume, compared to 2% of composition 2.

### Example 4 (comparison)

Similar to what described in Example 1, 50mL gel samples of the pharmaceutical composition for comparison (referred to as "composition 4") were prepared, totally similar to composition 1 of the invention described above, with the difference that composition 3 comprised a percentage of 3% by weight per volume of melatonin, as described in EP 2,702,992-A1, instead of 2% as in composition 1 of the present invention, and therefore beyond the maximum limit of 2.5% provided by the present invention. Evaluation of Composition 1 (invention)

5 patients with mucositis from polytherapy were examined. Mucositis was defined severe in a scale of severity of the disease, which starts from mild to cross the stages of average, severe and very severe intensity. The patients had pain, inflammation of the mucous membranes and dysphagia. These symptoms were such to jeopardize the normal diet; patients were often lacking appetite and reported pain on swallowing.

The patients were constantly administered 1 ml of sample of composition 1 of the invention (comprising 2% by weight per volume of melatonin) three times a day, for a week. The product was applied to the affected area by brush or the tip of the finger and left on site to act for a few minutes. Thereafter, the patient rinsed his mouth with water.

A gradual reduction of the symptoms manifested in patients before starting the therapy was observed, until complete elimination at the end of the week of treatment.

Also, during the period in which the patients were subjected to this treatment, no side effects were observed, such as numbness, tachycardia or depression.

### Evaluation of Composition 2 (invention)

Likewise, 5 patients with the same symptoms as mucositis from polypharmacy were examined.

The patients were constantly administered with 1 ml of sample of composition 2 of the invention (comprising 2% by weight per volume of melatonin and 0.12% by weight per volume of Chlorhexidine digluconate).

All patients showed a marked improvement of the clinical condition after only 3 days of therapy. Indeed, a marked regression of some symptoms was observed, in particular the elimination of dysphagia, buccal mucosal inflammation and pain, without at the same time having any negative side effects.

### Evaluation of Composition 3 (invention)

Likewise, 5 patients with the same symptoms as mucositis from polypharmacy were examined.

The patients were constantly administered with 1 ml of sample of composition 3 of the invention (comprising 1.8% by weight per volume of melatonin, 0.2% per weight per volume of essential lavender oil and 0.12% by weight per volume of Chlorhexidine).

Similar to the evaluation of composition 2 seen above, despite the lower amount of melatonin used, replaced in part by essential lavender oil, all patients showed the same gradual reduction of symptoms experienced in the patients before therapy, until complete elimination already after the third day of treatment.

### Evaluation of Composition 4 (comparison)

Likewise, 5 patients with the same symptoms as mucositis from polypharmacy were examined.

The patients were constantly administered with 1 ml of sample of composition 4 for comparison (comprising 3% by weight per volume of melatonin, beyond the maximum limit of 2.5% provided by the present invention).

All patients, albeit showing some improvement after 1 week of therapy, showed troublesome side-effects. A rise of the tendency to fall asleep, as well as an increased tachycardia and movement problems were thus observed.

Therefore, using a pharmaceutical composition of the invention which included a low concentration of melatonin, lower than 2.5% by weight per volume, very positive results were obtained in the treatment of mucositis, with no observed troublesome side effects, observed instead by administering a higher percentage of melatonin. The same positive results were obtained also by replacing part of melatonin (0.2% by weight per volume) with an essential lavender oil.

Moreover, using a pharmaceutical composition of the invention which included a low concentration of melatonin, lower than 2.5% by weight per volume, in combination with an amount, even if minimal (0.12% by weight per volume) of chlorhexidine digluconate, a synergistic effect of action of melatonin with chlorhexidine was obtained, generally with a considerable improvement of diseases of the mouth and mouth appendages, accelerating the disappearance time of symptoms in patients with mucositis.

The industrial invention has been described with reference to a preferred embodiment, but it will be understood that many modifications and variations will become apparent to the man skilled in the art, which may be made to such preferred embodiments described of the pharmaceutical composition and of its use according to the present invention, without departing from the granted scope of the invention itself.

Therefore, the breadth and scope of the present description should not be limited by any of the exemplary embodiments described above, but should be defined only on the basis of the following claims appended hereto and their equivalents.

## Claims

1. A pharmaceutical composition comprising a compound of general formula (I): 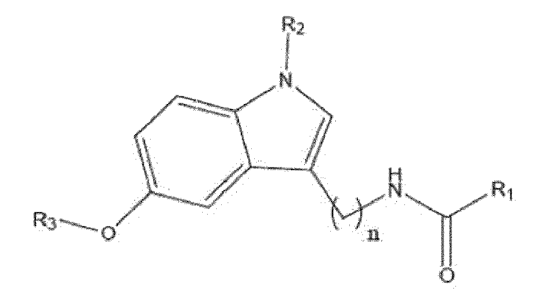 wherein n is an integer between 1 and 4;
R₁ and R₃, equal or different, represent a C₁-C₄ linear or branched alkyl group; and
R₂ is selected from hydrogen, a C₁-C₄ linear or branched alkyl group, a - C(=O) O-Ra group or a -C(=O)-N(H)-Ra group, wherein Ra is a C₁-C₄ linear or branched alkyl group,
or salts, prodrugs and/or solvates of such a compound of general formula (I), and chlorhexidine or a salt or derivative thereof.

2. A pharmaceutical composition according to claim 1, wherein R₁ and R₃, equal or different, are a C₁-C₂ alkyl group, preferably a methyl group, R₂ is hydrogen and n=1.

3. A pharmaceutical composition according to claim 1 or 2, wherein said compound of general formula (I) is melatonin.

4. A pharmaceutical composition according to any one of the preceding claims, wherein the concentration of melatonin is less than 2.5% by weight per volume of said pharmaceutical composition.

5. A pharmaceutical composition according to any one of the preceding claims, wherein the concentration of melatonin is in the range of 1.8%-2.2% by weight per volume of said pharmaceutical composition.

6. A pharmaceutical composition according to any one of the preceding claims, wherein the concentration of said chlorhexidine or a salt or derivative thereof is in the range from 0.01% to 0.50% by weight per volume of said pharmaceutical composition.

7. A pharmaceutical composition according to any one of the preceding claims, which comprises from about 1.8% to about 2.0% by weight per volume of melatonin and from about 0.10% to about 0.20% by weight per volume of chlorhexidine or a salt or derivative thereof of said pharmaceutical composition.

8. A pharmaceutical composition according to any one of the preceding claims, wherein said pharmaceutical composition further comprises lavender essential oil.

9. A pharmaceutical composition according to any one of the preceding claims, wherein said pharmaceutical composition is in gel form.

10. Use of a pharmaceutical composition according to any one of the preceding claims in the preparation of a medicament for treating and/or preventing mucositis.
